# FASCICULE DE BREVET EUROPEEN

(11) **EP 1 677 715 B1**
(45) Date de publication et mention de la délivrance du brevet: **20.02.2008**
(21) Numéro de dépôt: 04790248.1
(22) Date de dépôt: 11.10.2004
(51) Int. Cl.: A61F 7/02

(54) **COUSSIN THERMIQUE ET DISPOSITIF COMPRENANT UN TEL COUSSIN**
HEIZKISSEN UND EIN SOLCHES KISSEN ENTHALTENDE VORRICHTUNG
HEATING CUSHION AND DEVICE COMPRISING SUCH A CUSHION

(30) Priorité: 29.10.2003 EP 03024751
(43) Date de publication de la demande: 12.07.2006
(73) Titulaire: WELLCARE PRODUCTS S.A., 6970 Tenneville (BE)
(72) Inventeur: JOUHANNET, Guy, B-3724 Vliermaal (BE)
(74) Mandataire: pronovem
(86) Numéro de dépôt international: PCT/EP2004/011332
(87) Numéro de publication internationale: WO 2005/046540

(56) Documents cités:
- DE-A- 3 444 066
- FR-A- 1 018 835
- US-A- 5 069 208

## Description

### Domaine de l'invention

L'invention se rapporte aux coussins thermiques, ainsi qu'à leur utilisation.

### Etat de la technique

Le secteur hospitalier et la médecine ambulatoire font un usage important de coussins thermiques, notamment pour la régulation thermique externe et pour soulager la douleur ou dans diverses thérapies telles que la cryothérapie par exemple.

On sait en effet que l'application du froid peut soulager la douleur (maux de tête, migraine, maux de dents, douleurs musculaires ou inflammatoires, etc.) ou favoriser la résorption d'hématomes, d'oedèmes et la cicatrisation de plaies accidentelles ou chirurgicales.

Dans le brevet Etats-Unis US 3 545 230, on propose un coussin réfrigérant qui utilise la chaleur latente de fusion d'une substance solide. Durant cette phase de fusion, la température reste constante. Le coussin comprend une couche d'un gel hydrophile et insoluble, dans une enveloppe étanche et flexible. Un substrat inerte et flexible (par exemple des fibres ou un tissu), noyé dans le gel, sert à renforcer la résistance mécanique du coussin et à lui permettre d'épouser une forme géométrique quelconque. Pour conférer une flexibilité suffisante au coussin, on superpose plusieurs couches de gel de très faible épaisseur (quelques millimètres à peine) et on interpose un film flexible et inerte entre les couches de gels. Ce coussin connu présente le désavantage d'être de construction difficile et coûteuse, ses performances sont faibles et ses applications sont limitées.

Dans le document EP 0 123 949, la substance cryogénique du coussin consiste en des morceaux de gel de constitution particulière, présentant une élasticité comparable à celle du caoutchouc et ne collant pas entre eux à la température de production du froid. Le déplacement des morceaux de gel les uns par rapport aux autres dans le coussin confère à celui-ci la souplesse recherchée. Ces coussins connus ont toutefois le désagrément de nécessiter un gel coûteux et leur fabrication est délicate et coûteuse.

Dans le brevet Etats-Unis US 3 885 403, on suggère d'utiliser pour la substance cryogénique, un gel contenant une grande proportion d'un agent abaissant le point de congélation au-dessous de la température normale d'utilisation. Pour l'agent abaissant le point de congélation, on propose la glycérine et le propylène glycol. Avec ce coussin connu, la production du froid ne résulte pas d'une chaleur latente de fusion, mais du réchauffement progressif du gel. Ce coussin connu présente la propriété d'être de construction simple et de conserver une bonne flexibilité et une bonne aptitude à la déformation souple, du fait que le gel ne passe pas par une phase solide. Il présente par contre l'inconvénient d'une courte durée d'utilisation, puisque la production du froid ne résulte pas d'une chaleur latente de fusion, mais du réchauffement progressif du gel. En outre, la productivité du cousin (la production de froid ou l'extraction de calories par unité de temps) n'est pas constante mais diminue au fur et à mesure que le gel se réchauffe, ce qui constitue un désavantage supplémentaire de ce coussin connu.

Dans le document WO 97/11657, on décrit des panneaux alvéolaires formés d'un quadrillage de cellules remplies d'un agent thermique. Entre les cellules, le panneau présente des zones rectilignes à faible limite de rupture, qui permettent de le diviser à volonté (par cisaillement ou traction) en coussins thermiques de dimensions prédéterminées en fonction des applications auxquelles ces coussins sont destinés. Dans ces panneaux alvéolaires, les interstices sont la cause de plusieurs désavantages. D'une part, ils réduisent la capacité thermique du panneau et font d'autre part obstacle à une action homogène du panneau, lorsque celui-ci est appliqué sur la peau d'un patient.

Dans le document FR 1 018 835 A, on décrit des coussins thermiques comprenant un réseau de compartiments contenant une matière thermique et reliés entre eux par des membranes flexibles. Le réseau de compartiments est enfermé dans une enveloppe flexible formant, avec la paroi des compartiments, une enceinte qui est remplie d'un fluide thermique. Ces coussins thermiques connus présentent une piètre flexibilité et se prêtent mal à la déformation, principalement lorsque le fluide thermique de l'enveloppe est un liquide ou une matière solide à l'état de particules. Ils sont dès lors mal adaptés à une application uniforme sur un membre humain ou animal.

### Résumé de l'invention

L'invention vise à remédier aux désavantages énoncés plus haut des coussins thermiques connus et à répondre aux besoins des utilisateurs potentiels (notamment en milieu hospitalier), en fournissant un coussin thermique de conception nouvelle, qui concilie une excellente flexibilité/élasticité, est capable de s'adapter parfaitement même sur des formes en mouvement, avec un pouvoir calorifique élevé et durable à température sensiblement constante, dont l'action est homogène (notamment lorsqu'il est utilisé sur une partie du corps humain), dont l'épaisseur peut être maintenue sensiblement constante en cours d'utilisation, dont les dimensions et la forme sont sensiblement illimitées, qui peut subir des variations de pressions importantes sans se déformer, et qui, grâce aux propriétés énoncées plus haut, peut s'adapter à de multiples usages, notamment en milieu hospitalier, sans nécessiter des manipulations importantes.

En conséquence, l'invention concerne un coussin thermique comportant un réseau de blocs reliés par des articulations et séparés par des interstices, lesdits blocs comprenant une matière thermique et lesdits interstices étant comblés, au moins partiellement, avec une matière thermique déformable, ledit coussin thermique se caractérisant en ce que les articulations sont sélectionnées parmi
- les corps solides élastiques, qui sont fixés aux blocs et constituent alors une partie au moins de la matière thermique déformable susdite ;
- les membranes élastiques, qui sont fixées aux blocs et mises sous tension de manière à comprimer un corps déformable, présent dans les interstices, ledit corps déformable constituant alors une partie au moins de la matière thermique déformable ; et
- les articulations, qui sont perméables à un fluide présent dans les interstices, ledit fluide constituant alors une partie au moins de la matière thermique déformable.

Le coussin thermique selon l'invention est destiné à être mis en contact avec un corps matériel, dans le but d'influer sur les échanges de chaleur de ce corps avec le milieu ambiant.

L'expression « corps » doit être prise dans une acceptation générale, désignant un objet matériel. Elle désigne indifféremment un corps solide, un corps liquide ou un corps gazeux. Dans le cas d'un corps solide, celui-ci peut par exemple comprendre la surface d'un objet solide ou une partie de l'anatomie d'un animal ou d'un être humain. Dans le cas d'un corps liquide, celui-ci peut comprendre la surface d'une nappe ou d'un bain liquide. Dans cette application de l'invention, le coussin peut par exemple servir de cloison étanche entre deux liquides distincts ou entre un liquide et une atmosphère environnante, par exemple l'ambiance.
Dans le cas d'un corps gazeux, celui-ci comprend une atmosphère. Dans cette application de l'invention, le coussin selon l'invention peut par exemple former un écran entre deux atmosphères distinctes, par exemple une atmosphère chaude (l'atmosphère d'un four industriel) et une atmosphère à température modérée.
En variante, dans le cas d'un corps liquide ou gazeux, celui-ci peut éventuellement être enfermé dans une enveloppe étanche, par exemple une membrane souple.

Le milieu ambiant peut être un milieu gazeux ou un milieu liquide. Dans le cas d'un milieu gazeux, il peut être par exemple l'air atmosphérique, l'atmosphère d'un local chauffé, l'atmosphère d'un local réfrigéré (par exemple une chambre frigorifique) ou l'atmosphère d'un four industriel (liste non exhaustive). Dans le cas où le milieu ambiant est un milieu liquide, celui-ci peut par exemple être de l'eau ou un bain chimique industriel.

Dans la suite du présent mémoire, on désigne par l'expression « température normale d'utilisation », la température de la matière thermique des blocs et/ou des interstices, au moment de l'utilisation du coussin.

Dans la suite du présent mémoire, le mot « coussin » désigne de manière très générale tout élément solide et souple, destiné à être mis en contact avec le corps (tel que défini plus haut) pour le couvrir au moins partiellement ou l'envelopper totalement ou partiellement. Le coussin thermique selon l'invention peut dès lors être conformé de manière très diverse selon l'usage auquel on le destine. Il peut par exemple avoir la forme d'un objet de literie (oreiller, couverture, matelas), d'une poche, d'un manchon, d'un article vestimentaire ou d'une partie de vêtement, d'un emballage, d'un écran ou rideau, d'un revêtement (liste non exhaustive). Il peut être réalisé en modules divers assemblables.

Le coussin thermique selon l'invention est un coussin qui, lorsqu'il est mis en contact avec un corps (selon la définition donnée plus haut), à la température normale d'utilisation, engendre un transfert de chaleur entre ledit coussin et ledit corps ou concourt à maintenir la température dudit corps sensiblement constante.
Le coussin thermique selon l'invention est désigné « coussin calorifique » ou « coussin cryogénique » lorsque sa température normale d'utilisation est différente de celle du corps avec lequel on le met en contact, de telle sorte qu'un transfert de chaleur s'opère entre ledit coussin et ledit corps. Lorsque le transfert de chaleur s'opère naturellement dans le sens du corps vers le coussin thermique, celui-ci est un coussin cryogénique. Dans le cas où le transfert de chaleur s'opère dans le sens du coussin thermique vers le corps, le coussin thermique selon l'invention est un coussin calorifique.
Le coussin thermique selon l'invention est désigné « coussin isothermique » lorsque sa température normale d'utilisation est sensiblement égale à celle du corps avec lequel on le met en contact, de telle sorte qu'il n'y ait pas de transfert de chaleur entre ledit corps et le coussin thermique. Un coussin isothermique conforme à l'invention exerce dès lors la fonction technique d'un isolant calorifique, en faisant obstacle à un transfert de chaleur entre le corps et le milieu ambiant.

Le coussin thermique selon l'invention comprend un réseau de blocs, entre lesquels sont ménagés des interstices. Les interstices communiquent généralement entre eux.

Par définition, chaque bloc du coussin est un élément solide. La forme des blocs du coussin selon l'invention sera détaillée plus loin.

Les blocs sont assemblés en réseau. Ils sont par ailleurs espacés de manière à former entre eux un réseau d'interstices. Le coussin selon l'invention comprend de la sorte un entrelacs d'interstices dans un entrelacs de blocs. Le réseau de blocs comprend généralement une seule couche ou strate de blocs, bien qu'un réseau formé de plusieurs strates de blocs superposées ne soit pas exclu de l'invention.

Les blocs du réseau sont articulés entre eux. En d'autres termes, les blocs du réseau sont reliés entre eux par des articulations. Des détails concernant les articulations seront fournis plus loin.

Dans le coussin selon l'invention, la forme et les dimensions des blocs, la forme et les dimensions des interstices et le choix des articulations sont adaptés pour que le réseau de blocs forme une structure solide, déformable par flexion et/ou torsion et/ou rotation autour d'axes multiples répartis dans un espace à trois dimensions. Cette particularité du coussin selon l'invention lui permet de s'adapter facilement à pratiquement n'importe quelle forme de corps sur lequel on l'applique (par exemple une partie de l'anatomie humaine ou animale) et d'en accompagner le mouvement.

Dans le coussin selon l'invention, les blocs et les interstices contiennent une matière thermique. La matière thermique sera explicitée plus loin.

La forme des blocs dépend de l'usage du coussin et n'est pas critique pour la définition de l'invention. Les blocs peuvent par exemple avoir une forme sphérique, hémisphérique, ovoïde, annulaire, lenticulaire, conique, tronconique ou polyédrique. Les blocs peuvent avoir des faces planes, cintrées ou gauches, par exemple hélicoïdales ou comprendre un assemblage de faces planes et de faces cintrées ou gauches. Ils peuvent par exemple avoir la forme de tonnelets qui associent une face annulaire cintrée et des faces planes aux extrémités. De manière générale, toute forme convient, qui, associée aux interstices et aux articulations liant les blocs, permet une déformation du coussin comme explicité plus haut. Les formes polyédriques sont généralement préférées. Parmi les formes polyédriques, on préfère les polyèdres droits ou pyramidaux. Les pyramides tronquées sont spécialement recommandées. Parmi les polyèdres droits ou pyramidaux, on préfère les polyèdres à base triangulaire, trapézoïdale, carrée, rectangulaire ou octogonale.

Dans une forme de réalisation préférée de l'invention, chaque bloc est formé de deux troncs de pyramide joints le long de leur grande base, par exemple deux troncs de pyramide triangulaire, trapézoïdale, carrée ou octogonale. Dans cette forme de réalisation préférée de l'invention, les blocs sont avantageusement articulés dans le plan géométrique de la grande base susdite des deux troncs de pyramide.

Comme exposé plus haut, les interstices ménagés entre les blocs ont pour fonction de permettre un déplacement de ces blocs autour des articulations qui les lient. La forme des interstices va dépendre de divers paramètres, tels que le type de déformations souhaitées pour le réseau de blocs (flexion uniaxiale, biaxiale ou triaxiale ; torsion ; extension ; ou combinaison de deux ou plusieurs de ces types de déformations), l'amplitude des déformations, la forme et les dimensions des blocs, les articulations utilisées et la disposition de ces articulations entre les blocs.

Dans le coussin thermique selon l'invention, les blocs comprennent une matière thermique. La matière thermique des blocs confère ses propriétés thermiques au coussin thermique selon l'invention.
Dans le cas d'un coussin cryogénique, la matière thermique des blocs est sélectionnée parmi celles qui, portées à la température normale d'utilisation et soumises ensuite à un refroidissement, libèrent une quantité de chaleur importante.
Dans le cas d'un coussin calorifique, la matière thermique des blocs est sélectionnée parmi celles qui, portées à la température normale d'utilisation et soumises ensuite à un chauffage, captent une quantité de chaleur importante.
Dans le cas d'un coussin isothermique, la matière thermique des blocs est sélectionnée parmi celles qui s'opposent au transfert de chaleur ou qui, portées à la température normale d'utilisation, nécessitent ensuite un apport important de chaleur pour modifier sa température.
Les matières thermiques possédant, à la température normale d'utilisation, une chaleur spécifique élevée conviennent en général pour les trois types de coussins selon l'invention. Ces matières thermiques sont celles connues et utilisées en technique pour leur propriété d'accumuler des quantités importantes de chaleur et qui trouvent notamment des applications en tant qu'accumulateurs thermiques.

Dans le coussin thermique selon l'invention, les blocs peuvent être entièrement constitués par la matière thermique qui est alors nécessairement solide à la température normale d'utilisation, ainsi que dans les conditions normales de manutention du coussin thermique. En variante, les blocs peuvent, en plus de la matière thermique, comprendre une autre matière, qui ne réalise pas, à elle seule, la fonction de la matière thermique telle que définie plus haut.
La matière thermique peut être identique pour tous les blocs ou être différente selon les blocs. Par ailleurs, chaque bloc peut comprendre une seule matière thermique ou un mélange de deux ou plusieurs matières thermiques. Dans le présent mémoire, l'expression « matière thermique » désigne indifféremment une matière thermique unique ou un mélange de matières thermiques distinctes.

Dans une forme de réalisation particulière du coussin thermique selon l'invention, les blocs comprennent des cellules à l'intérieur desquelles est disposée la matière thermique. Cette forme de réalisation du coussin thermique selon l'invention est spécialement bien adaptée au cas où la matière thermique des blocs n'est pas solide à la température normale d'utilisation ou dans les conditions normales de manutention du coussin thermique. Dans cette forme de réalisation du coussin selon l'invention, la forme extérieure des cellules doit respecter les conditions de forme exposées plus haut en ce qui concerne les blocs. Le choix du matériau des cellules est conditionné par la nécessité, pour lesdites cellules, d'être chimiquement inertes vis-à-vis de la matière thermique qu'elles contiennent et vis-à-vis de l'environnement chimique et thermique pendant leur utilisation normale. Les cellules doivent par ailleurs posséder des propriétés mécaniques compatibles avec les sollicitations mécaniques auxquelles le coussin est normalement soumis à la température normale d'utilisation ou en cours de manutention et être sensiblement indéformables (elles doivent être en un matériau rigide ou semi-rigide et de forme adaptée). Dans ce cas particulier, chaque cellule peut être entièrement isolée des cellules voisines ou, en variante, les cellules peuvent communiquer entre-elles, pour permettre au contenu fluide desdites cellules de circuler entre celles-ci. Dans le cas où la matière thermique du coussin est liquide ou gazeuse, la paroi des cellules est généralement imperméable aux liquides ou aux gaz. Les matières plastiques conviennent généralement bien pour la constitution des cellules. Des matières plastiques utilisables comprennent les polyoléfines, particulièrement les polymères et les copolymères de l'éthylène et du propylène, les polymères chlorés, particulièrement les polymères et les copolymères du chlorure de vinyle et du chlorure de vinylidène, les copolymères styrène-éthylène-butyle-styrène et le polyuréthane. Le polyéthylène, le polypropylène, le polychlorure de vinyle et les copolymères styrène-éthylène-butyle-styrène conviennent bien dans la majorité des applications. En variante, les cellules peuvent également être réalisées en métal, par exemple par emboutissage de feuilles de métal ou d'alliage malléable tels que l'aluminium et les alliages d'aluminium. Selon une autre variante, les cellules ou certaines d'entre-elles sont formées de matières composites : une partie de la cellule est en une matière et une autre partie de la cellule est réalisée en une autre matière. Selon une variante supplémentaire, une partie du réseau de cellules sont réalisées en une matière et une autre partie du réseau de cellules sont réalisées en une autre matière.

Tout procédé connu de mise en forme peut être utilisé pour fabriquer le réseau de cellules. Les procédés par moulage, emboutissage, extrusion, injection, conviennent bien.
Selon une méthode adéquate, on forme une feuille gaufrée en matière plastique ou en métal, qui présente ainsi un réseau d'alvéoles ou semi-cellules. On colle ou soude ensuite une autre feuille sur la feuille gaufrée, de manière à obturer les alvéoles de la feuille gaufrée et former les cellules. Cette autre feuille peut être une feuille unie ou une feuille gaufrée.

Dans une forme de réalisation préférée de l'invention, la matière thermique des blocs comprend une substance qui subit un changement d'état à la température normale d'utilisation. Dans cette forme de réalisation de l'invention, l'expression « changement d'état » est considérée dans son acceptation générale et désigne tout changement physique et/ou chimique de la substance, se produisant à une température sensiblement constante et caractérisé par une chaleur latente de changement d'état. Selon la substance utilisée, le changement d'état peut notamment comprendre la fusion d'un solide, la solidification d'un liquide, la vaporisation d'un liquide, la condensation d'un gaz, l'hydratation totale ou partielle d'un sel, la déshydratation totale ou partielle d'un sel, la cristallisation d'un solide amorphe ou la recristallisation d'une forme allotropique d'un cristal dans une autre forme allotropique. Dans cette forme de réalisation de l'invention, la matière thermique des blocs peut être exclusivement constituée par ladite substance qui subit un changement d'état à la température normale d'utilisation. En variante, la matière thermique des blocs peut en outre contenir d'autres substances ou matériaux qui ne subissent pas un changement d'état à la température normale d'utilisation. Par la suite, pour éviter de surcharger inutilement le texte, on supposera que c'est la matière thermique tout entière qui subit un changement d'état à la température normale d'utilisation. Il est toutefois bien entendu que, comme exposé plus haut, l'invention n'exclut pas le cas où seule une partie de la matière thermique des blocs subit un changement d'état (en l'occurrence, lorsqu'elle est composée d'un mélange de plusieurs composés chimiques dont un seul subit un changement d'état à la température normale d'utilisation ou lorsqu'on y incorpore un matériau additionnel qui ne participe pas à la fonction thermique du coussin thermique, par exemple un aimant).

Dans la forme de réalisation préférée définie ci-dessus, le changement d'état subi par la matière thermique des blocs à la température normale d'utilisation correspond à une absorption de chaleur par ladite matière dans le cas d'un coussin cryogénique et à une cession de chaleur dans le cas d'un coussin calorifique.
Dans le cas d'un coussin isothermique, le changement d'état subi par la matière thermique des blocs à la température normale d'utilisation correspond à une absorption de chaleur par ladite matière thermique lorsque ladite température normale d'utilisation (qui est aussi celle du corps auquel on destine le coussin) est inférieure à celle du milieu ambiant. Le changement d'état correspond à une cession de chaleur par la matière thermique, lorsque la température normale d'utilisation est supérieure à celle du milieu ambiant.

Pour utiliser un coussin thermique conforme à la forme de réalisation préférée qui vient d'être décrite, il convient de sélectionner convenablement l'état de la matière thermique des blocs. Dans le cas où on utilise le coussin en tant que coussin cryogénique, il est nécessaire d'amener d'abord la matière thermique des blocs dans un état qui, à la température normale d'utilisation va subir un changement d'état correspondant à une absorption de chaleur (par exemple la fusion d'un solide ou la vaporisation d'un liquide). Dans le cas où on utilise le coussin en tant que coussin calorifique, il est nécessaire d'amener d'abord la matière thermique des blocs dans un état qui, à la température normale d'utilisation va subir un changement d'état correspondant à une production de chaleur (par exemple la solidification ou la cristallisation d'un liquide ou la condensation d'un gaz). Dans le cas où on utilise le coussin en tant que coussin isothermique, dans un milieu ambiant dont la température est supérieure à la température normale d'utilisation, il est nécessaire d'amener d'abord la matière thermique des blocs dans un état qui, à la température normale d'utilisation va subir un changement d'état correspondant à une absorption de chaleur (par exemple la fusion d'un solide ou la vaporisation d'un liquide). Dans le cas où on utilise le coussin en tant que coussin isothermique, dans un milieu ambiant dont la température est inférieure à la température normale d'utilisation, il est nécessaire d'amener d'abord la matière thermique des cellules dans un état qui, à la température normale d'utilisation va subir un changement d'état correspondant à une production de chaleur (par exemple la solidification ou la cristallisation d'un liquide ou la condensation d'un gaz. Toutes autres choses étant égales par ailleurs, dans la forme de réalisation préférée qui vient d'être décrite, les meilleurs résultats sont obtenus avec des matières thermiques qui, à la température normale d'utilisation, possèdent une chaleur latente de changement d'état élevée.

Dans la forme de réalisation préférée qui vient d'être décrite, la matière thermique des blocs, subissant un changement d'état à la température normale d'utilisation peut être un corps pur. En variante, elle peut être une composition chimique qui est congruente à ladite température normale d'utilisation, de manière que le changement d'état s'opère à une température sensiblement constante.

Dans la forme de réalisation préférée décrite ci-dessus et ses variantes de réalisation, le choix de la matière thermique des blocs est conditionné par la température normale d'utilisation du coussin. Celle-ci est elle-même conditionnée par l'application à laquelle on destine le coussin thermique. L'eau et les solutions aqueuses conviennent généralement bien dans le cas particulier d'un coussin cryogénique destiné à des applications thérapeutiques. L'eau pure convient bien dans le cas d'applications où la température normale d'utilisation du coussin est voisine de 273 K (0 °C). Pour des applications où la température normale d'utilisation est inférieure à 273 K, on préconise des solutions aqueuses dans lesquelles le corps dissous et sa concentration sont sélectionnés en fonction de la température normale d'utilisation du coussin cryogénique. On recommande de sélectionner le corps dissous et sa concentration de manière à éviter une précipitation partielle ou totale dudit corps dissous à la température normale d'utilisation du coussin. Des exemples de corps dissous comprennent le chlorure de sodium, le chlorure de calcium, le carbonate de sodium, le propylène glycol, la glycérine, l'alcool éthylique et l'alcool propylique.

Comme exposé plus haut, lorsque la matière thermique des blocs est liquide, celle-ci est enfermée dans des cellules dont la paroi est généralement imperméable aux liquides. Lorsqu'on utilise une matière thermique liquide qui subit une vaporisation à la température normale d'utilisation, il peut se révéler avantageux d'utiliser, pour les cellules, une paroi à perméabilité orientée ou une paroi qui est imperméable à la phase liquide de la matière thermique, mais perméable à sa phase gazeuse (par exemple PU imper-respirant). L'invention n'est toutefois pas limitée à cette forme de réalisation et couvre également le cas où la paroi des cellules est imperméable à la phase liquide et à la phase gazeuse de la matière thermique des cellules.

Dans le coussin thermique selon l'invention, la matière thermique des blocs constitue l'élément actif principal du coussin et lui confère ses propriétés thermiques (propriétés cryogéniques, calorifiques ou isothermiques, selon sa destination).

La matière thermique des interstices a pour fonction d'accroître l'efficacité du coussin, en augmentant sa surface active et son volume actif. La matière thermique des interstices doit posséder des propriétés thermiques analogues à celles énoncées plus haut pour la matière thermique des blocs. Ses propriétés thermiques doivent dès lors être adaptées à la destination du coussin, à la température normale d'utilisation de celui-ci, à la température du corps auquel on destine le coussin et à la température ambiante. Pour ce qui concerne les propriétés thermiques de la matière thermique des interstices, on peut par conséquent répéter ce qui a été exposé plus haut pour la matière thermique des blocs.

La matière thermique des interstices doit par ailleurs être déformable. Cette propriété additionnelle de la matière thermique des interstices est nécessaire pour permettre le mouvement des blocs articulés et la déformation du coussin. Le choix de la matière thermique des interstices et/ou son mode de mise en oeuvre sont dès lors conditionnés par les paramètres constructifs du coussin, tels que la forme des blocs, la forme des interstices séparant les blocs, les articulations des blocs et la position de ces articulations dans le réseau de blocs.

Dans une forme de réalisation particulière du coussin thermique selon l'invention, la matière thermique des interstices comprend un corps solide élastique. Celui-ci peut par exemple comprendre une mousse en polymère de synthèse ou un élastomère, par exemple du caoutchouc naturel ou synthétique. Dans le cas d'une mousse, celle-ci peut être du type à pores ouverts ou du type à pores fermés. Dans le cas d'une mousse à pores fermés, ces pores peuvent être occupés par un gaz dont le coefficient de transmission de chaleur est inférieur à celui de l'air, par exemple de l'azote ou de l'argon.

Dans une autre forme de réalisation du coussin thermique selon l'invention, la matière thermique des interstices comprend un fluide. Le fluide peut comprendre un liquide, un gaz, un gel (ou fluide visqueux) ou un solide à l'état de particules meubles (par exemple une poudre). Dans cette forme de réalisation de l'invention, les interstices doivent être obturés pour y retenir la matière thermique. L'organe d'obturation utilisé ne doit pas faire obstacle au mouvement des blocs sur leurs articulations. Des informations complémentaires concernant l'organe d'obturation des interstices seront fournies plus loin.

Dans le coussin thermique selon l'invention, les interstices peuvent être comblés partiellement ou totalement par la matière thermique. On préfère que les interstices du réseau de blocs soient comblés totalement avec la matière thermique.

En fonction des applications auxquelles le coussin thermique selon l'invention est destiné, des substances susceptibles de réfléchir le rayonnement infrarouge ou le rayonnement ultraviolet peuvent éventuellement être incorporées aux matières thermiques des blocs et des interstices et/ou, le cas échéant, aux membranes ou enveloppes. Des exemples de telles substances réfléchissantes comprennent notamment des poudres d'aluminium. De manière similaire, pour d'autres applications particulières, on peut incorporer aux matières thermiques et/ou, le cas échéant, aux membranes ou enveloppes, des substances susceptibles d'absorber le rayonnement infrarouge, par exemple de la poudre de carbone.

Dans le coussin thermique selon l'invention, les interstices entre les blocs peuvent être ouverts. Cette forme de réalisation de l'invention convient lorsque la matière thermique déformable des interstices est un solide déformable (par exemple une mousse) à la température normale d'utilisation et dans les conditions normales de manutention du coussin thermique.
De manière alternative, les interstices entre les blocs peuvent être obturés par un organe d'obturation. Cette alternative s'impose dans le cas où la matière thermique des interstices est un fluide (tel que défini plus haut) dans les conditions normales d'utilisation et de manutention du coussin thermique. Elle convient aussi dans le cas où la matière thermique des interstices est un corps solide déformable. Comme énoncé plus haut, il convient de choisir un organe d'obturation qui ne fasse pas obstacle au mouvement des blocs articulés.

Le coussin thermique selon l'invention se particularise par la conception des articulations des blocs. Il se particularise spécialement par une sélection originale desdites articulations, liée à une sélection de la matière thermique des interstices, de manière que le réseau de blocs puisse subir des déformations dans les trois directions de l'espace, par flexion, torsion et rotation.

En conséquence, selon un premier mode d'exécution de l'invention, les articulations des blocs comprennent des corps solides élastiques, qui sont fixés aux blocs et qui constituent alors une partie au moins de la matière thermique des interstices. Des informations concernant le corps solide élastique ont été communiquées plus haut.

Dans ce premier mode d'exécution de l'invention, la fixation du corps solide élastique aux blocs peut être réalisée par tous moyens adéquats, par exemple par collage, par soudage ou au moyen de vis ou de chevilles (liste non exhaustive).

Dans un deuxième mode d'exécution de l'invention, les articulations des blocs comprennent des membranes élastiques, qui sont fixées aux blocs et qui sont mises sous tension de manière à comprimer un corps déformable, présent dans les interstices, ledit corps déformable constituant alors une partie au moins de la matière thermique déformable. Le corps déformable peut être un corps solide élastique ou un fluide. Dans le cas d'un fluide, celui-ci peut être un fluide incompressible ou un fluide compressible. Des informations concernant les corps solides élastiques et les fluides ont été communiquées plus haut.

Dans ce mode d'exécution de l'invention, chaque interstice peut être recouvert d'une membrane individuelle. Les membranes sont alors fixées de manière appropriée aux blocs pour permettre leur mise sous tension. Tous moyens appropriés peuvent être mis en oeuvre, par exemple un collage ou un soudage. La fixation des membranes aux blocs doit être étanche, lorsque la matière thermique des interstices est fluide (gaz ou liquide).
Dans une variante particulière du deuxième mode d'exécution susdit de l'invention, une feuille étanche et élastique recouvre une partie au moins ou la totalité du réseau de blocs et elle se substitue alors aux membranes individuelles obturant les interstices.
Dans une autre variante particulière du deuxième mode d'exécution susdit de l'invention, le réseau de blocs est enfermé dans une enveloppe étanche et élastique. Dans cette autre variante, l'enveloppe peut être fixée de manière appropriée aux blocs pour obturer les interstices de manière étanche lorsque la matière thermique des interstices est fluide (gaz ou liquide). Dans le cas où la matière thermique des interstices est un solide déformable (par exemple une mousse ou un élastomère), l'enveloppe peut indifféremment être fixée à tous les blocs du réseau de blocs, à certains de ceux-ci (et pas aux autres) ou n'être fixée à aucun bloc. La fixation de l'enveloppe aux blocs peut être obtenue par tous moyens appropriés. Des moyens appropriés comprennent le collage et le soudage. L'enveloppe peut être simple ou constituée d'un film complexe ou comporter plusieurs couches liées entre-elles ou non. Elle peut être de composition distincte selon la face. Elle peut comporter des traitements et additifs qui lui confèrent des propriétés actives spécifiques selon l'utilisation. Elle peut comporter des tubulures d'entrée/sortie pour l'introduction ou la circulation de la matière thermique des interstices, lorsque celle-ci est fluide.

Dans un troisième mode d'exécution de l'invention, les interstices entre les blocs contiennent un fluide et les articulations sont perméables audit fluide. Dans ce mode d'exécution de l'invention, le fluide peut comprendre un liquide, un gaz, un gel ou un solide à l'état de particules meubles (par exemple une poudre) et il constitue une partie au moins de la matière thermique déformable des interstices. Des informations concernant le fluide des interstices ont été communiquées plus haut. Dans ce troisième mode d'exécution de l'invention, la sélection des articulations va dépendre de l'état du fluide présent dans les interstices. Lesdites articulations peuvent par exemple comprendre des membranes flexibles (éventuellement élastiques), poreuses aux gaz ou aux liquides, des cloisons flexibles et perforées, des treillis, des charnières présentant des perforations ou tout autre articulation susceptible de ne pas entraver une libre circulation du fluide présent dans les interstices.

En fonction du type de matière thermique déformable présente dans les interstices, le coussin selon l'invention peut associer plusieurs types d'articulations, par exemple des corps solides élastiques fixés aux blocs, des pivots et des membranes.
En variante, en plus des articulations, les blocs du réseau peuvent être reliés entre eux par des tenons rigides, dont la résistance mécanique à la flexion est faible et contrôlée. Dans cette variante de l'invention, les tenons rigides servent à conférer une rigidité au coussin pour faciliter sa manutention et elles se brisent dès que l'on applique le coussin sur le corps et le déforme.

Le coussin thermique selon l'invention présente la propriété avantageuse de combiner d'excellentes propriétés de stockage et d'échange thermique et une excellente flexibilité/élasticité qui permet de l'adapter immédiatement à la forme d'un corps matériel sur lequel on l'applique, de telle sorte qu'il vienne épouser parfaitement la forme de ce corps et assure alors une régulation homogène de la température ou des échanges thermiques sur toute la surface couverte dudit corps. Du fait de la conception et de la structure du coussin thermique selon l'invention, l'épaisseur de celui-ci est sans effet sur sa flexibilité et son aptitude à la déformation. En d'autres termes, l'invention permet d'adapter l'épaisseur du coussin aux propriétés thermiques recherchées (à sa capacité thermique), sans nuire à sa flexibilité ni à son aptitude à s'adapter facilement à des corps de formes diverses.

Dans une forme de réalisation particulière du coussin thermique selon l'invention, le réseau de blocs porte, sur une partie au moins de sa surface, une enveloppe élastique et étanche, généralement munie d'un dispositif d'admission et d'échappement d'un fluide (par exemple un gaz). Dans cette forme de réalisation de l'invention, l'enveloppe est destinée à être mise en contact avec le corps matériel précité, avec lequel doit s'opérer un échange de chaleur. Par l'introduction d'un volume défini d'un fluide approprié dans l'enveloppe (généralement de l'air), il est possible de réguler le transfert de chaleur entre le coussin thermique et le corps matériel.

Dans le coussin thermique selon l'invention, le réseau de blocs peut être conformé en une strate unique. En variante, le réseau de blocs peut être formé d'une superposition de plusieurs strates. La strate ou l'ensemble stratifié peut avantageusement être pris en sandwich entre deux enveloppes élastiques et étanches, généralement munies chacune d'un dispositif d'admission et échappement d'un fluide (par exemple un gaz). Dans cette forme de réalisation de l'invention, l'une des enveloppes sert alors, en prenant appui sur un support adéquat (par exemple un bandage ou une coquille), à appliquer le coussin thermique sur le corps avec une pression définie. L'autre enveloppe sert à réguler le transfert de chaleur entre les matières thermiques et le corps, comme exposé plus haut.

Le coussin thermique selon l'invention trouve de multiples applications. Il trouve notamment une utilisation en tant que coussin isothermique (ou thermostat) pour maintenir la température de récipients tels que des bouteilles ou des flacons thermostatiques. Une telle utilisation du coussin thermique selon l'invention trouve des applications dans l'industrie pour le maintien de produits chimiques à des températures prédéterminées, ainsi que dans le secteur alimentaire pour réchauffer, refroidir ou conserver la température d'enceintes contenant des denrées alimentaires.

Le coussin thermique selon l'invention trouve également des applications en tant que coussin cryogénique, notamment dans l'industrie chimique ou pharmaceutique pour le maintien de produits chimiques à de basses températures, ainsi que dans l'industrie alimentaire pour préserver la conservation de denrées alimentaires.

Du fait de son caractère moulant et déformable, le coussin thermique convient particulièrement aux applications liées au bien-être tant sous forme d'articles vestimentaires que d'articles de soins corporels divers (par exemple : compresses, masques, outils de massage).

Du fait de son excellente aptitude à la déformation, le coussin thermique selon l'invention est spécialement adapté aux applications médicales, paramédicales et sportives, notamment en régulation thermique et en cryothérapie. La cryothérapie est une technique médicale qui est largement utilisée, notamment pour soulager la douleur (par exemple les maux de tête, les migraines, les maux de dents, les douleurs musculaires ou les douleurs inflammatoires), pour faciliter la résorption d'hématomes ou d'oedème et la cicatrisation de plaies accidentelles ou chirurgicales.

L'invention concerne dès lors également un dispositif comprenant un coussin thermique conforme à l'invention, pour le traitement thérapeutique du corps humain ou animal.

Dans le dispositif selon l'invention, le coussin et son réseau de blocs sont conformés en fonction du mode de traitement ou en fonction de la partie du corps humain ou du corps animal à laquelle on destine ledit dispositif.

Dans une forme de réalisation particulière du dispositif selon l'invention, le coussin est logé à l'intérieur d'une coquille rigide. Dans cette forme de réalisation du dispositif selon l'invention, la coquille est normalement conformée en fonction de la partie du corps humain ou animal sur laquelle le coussin doit être appliqué. Elle a par exemple la forme d'un coude, d'un genou, d'un doigt, d'un pied ou d'un crâne.

Dans cette forme de réalisation du dispositif selon l'invention, la coquille peut être formée de deux ou plusieurs éléments articulés, pour faciliter sa mise en place sur le corps humain ou animal. Ce mode de réalisation du dispositif selon l'invention est spécialement indiqué lorsque le dispositif est destiné à être appliqué sur la tête d'une personne ou d'un animal.

Dans une variante avantageuse de la forme de réalisation qui vient d'être décrite, une enveloppe élastique et étanche est interposée entre la coquille et le réseau de blocs du coussin et cette enveloppe est généralement munie d'un dispositif d'admission et échappement d'un fluide. Cette variante de l'invention réalise une application homogène du coussin thermique sur le corps humain et animal. Elle permet en outre d'appliquer le coussin thermique sur le corps humain ou animal avec une pression définie, réglée par la pression du fluide admis dans l'enveloppe.

Dans une autre variante de la forme de réalisation décrite plus haut, un panneau est articulé à la coquille et dimensionné de telle sorte qu'il puisse former, avec la coquille, une chambre hermétique contenant le coussin thermique. Dans cette variante du dispositif selon l'invention, le coussin thermique et la coquille font partie intégrante d'un coffret hermétique servant à la manutention du dispositif. En utilisant un panneau convenablement isolé, le dispositif ainsi conçu permet de conserver des propriétés thermiques prédéterminées. De la sorte, le dispositif est spécialement bien adapté à une utilisation en urgence, en dehors d'un centre hospitalier, par exemple sur la voie publique.
Dans la variante susdite du dispositif selon l'invention, la coquille et le panneau articulé peuvent être munis d'une instrumentation médicale, comprenant par exemple des thermomètres ou des tensiomètres. Elle peut également être munie d'un appareil autonome pour générer de la chaleur ou du froid, pendant la manutention du dispositif, notamment son transport ou son stockage.

L'invention concerne également une paroi thermique, comprenant une accumulation de coussins thermiques conformes à l'invention, entre deux cloisons ou entre une cloison et un corps. La paroi thermique peut être une paroi calorifique, une paroi cryogénique ou une paroi isostatique, selon que les coussins thermiques accumulés entre ses deux cloisons sont des coussins calorifiques, des coussins cryogéniques ou des coussins isostatiques.

La paroi thermique selon l'invention peut par exemple constituer la paroi d'un thermos.

Dans la paroi thermique selon l'invention, on utilise de préférence des coussins thermiques dans lesquels le réseau de blocs est disposé dans une enveloppe comblée d'une matière thermique déformable. Une partie au moins de la matière thermique de l'enveloppe peut comprendre la matière thermique des interstices.

### Brève description des figures

Des particularités et détails de l'invention vont apparaître au cours de la description suivante des figures annexées, qui représentent quelques formes de réalisation particulière de l'invention.
La figure 1 est une vue schématique, en section transversale, d'une forme de réalisation particulière du coussin thermique selon l'invention ;
La figure 2 montre en perspective, avec arrachement partiel, un détail du coussin thermique de la figure 1 ;
La figure 3 montre en perspective un détail de la figure 2 ;La figure 4 montre, en section horizontale, une autre forme de réalisation du coussin thermique selon l'invention ;
La figure 5 montre le coussin thermique de la figure 4, en section verticale ;
La figure 6 montre en perspective une forme de réalisation particulière du dispositif selon l'invention ;
La figure 7 est une vue latérale, avec arrachement partiel, d'une autre forme de réalisation du dispositif selon l'invention ;
La figure 8 est une vue arrière du dispositif de la figure 7 ;
La figure 9 est un schéma, en section transversale, d'une troisième forme de réalisation du dispositif selon l'invention ;
La figure 10 montre en section tranversale verticale une paroi thermique conforme à l'invention ;
La figure 11 montre un coussin thermique conforme à l'invention, utilisable dans la paroi thermique de la figure 10 ; et
La figure 12 montre un autre coussin thermique conforme à l'invention, utilisable dans la paroi thermique de la figure 10.

Les figures ne sont pas dessinées à l'échelle.

Généralement, les mêmes numéros de référence désignent les mêmes éléments.

### Description détaillée de modes de réalisation particuliers

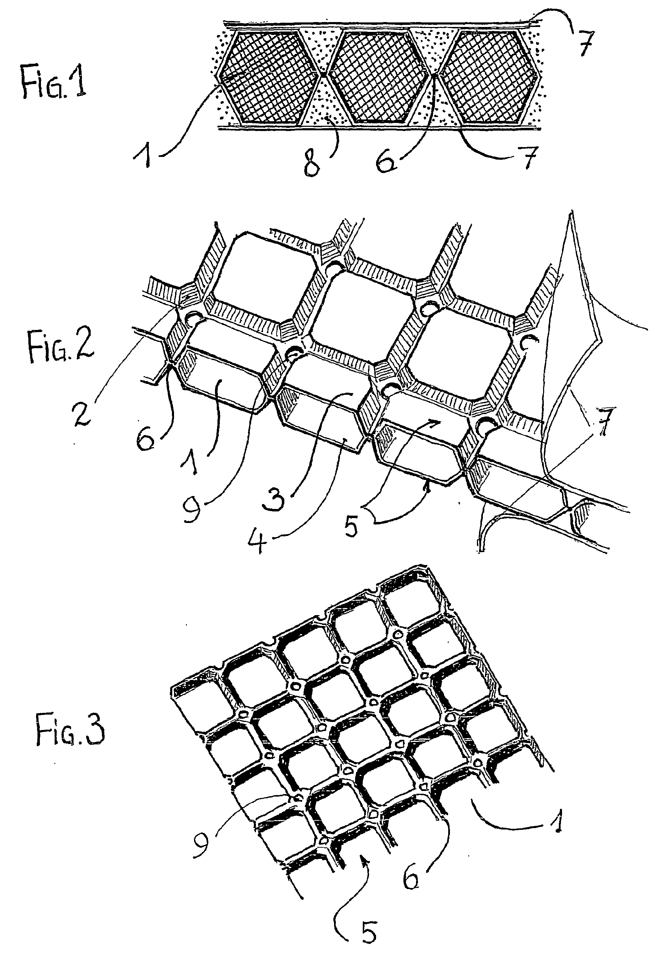

Le coussin thermique représenté aux figures 1 et 2 comprend un réseau de blocs 1, formés de cellules creuses. Les blocs ou cellules 1 ont la forme de prismes à base carrée. Chaque cellule 1 est formée de deux troncs de pyramides 3 et 4, raccordés le long de leur grande base carrée. Les arêtes latérales des deux troncs de pyramide sont chanfreinées (2). Le réseau des cellules 1 est formé de l'assemblage de deux feuilles gaufrées 5 en matière polymérique souple (par exemple en polychlorure de vinyle plastifié ou en copolymère styrène-éthylène-butyle-styrène) préalablement embouties et soudées l'une à l'autre le long de languettes 6 entre les cellules (une feuille gaufrée 5 est représentée à la figure 3). Les languettes 6 sont souples et élastiques et elles servent d'articulations entre les cellules 1. Elles sont percées d'ouvertures 9 dont la fonction apparaîtra plus loin. En pliant ou tordant l'assemblage des feuilles 5, on peut ainsi faire adopter des profils complexes au coussin de cellules, pour l'adapter à un corps approprié, par exemple une partie de l'anatomie humaine ou animale.

Le coussin thermique des figures 1 à 3 est un coussin cryogénique, destiné à une cryothérapie. A cet effet, les cellules 1 sont remplies d'une matière thermique appropriée, dont une définition a été donnée plus haut. La matière thermique est avantageusement une solution aqueuse dont la congélation est congruente et a lieu à une température correspondant à la température à laquelle le coussin est destiné (température normale d'utilisation). Lorsque la température normale d'utilisation est voisine de 273 K (ou 0°C), la solution aqueuse peut être remplacée par de l'eau distillée ou minéralisée.

Les interstices 8 délimités entre les cellules 1 et les languettes 6 sont comblés avec une matière thermique déformable 10(représentée à la figure 1 uniquement). La matière thermique 10 représentée à la figure 1 est un corps solide élastique, par exemple une masse en élastomère ou une mousse polymérique. Elle est de préférence choisie de manière à présenter une chaleur spécifique élevée à la température normale d'utilisation.

Une enveloppe souple et élastique 7 entoure le réseau des cellules 1 et d'interstices 8. L'enveloppe élastique 7 est par exemple un film en copolymère styrène-éthylène-butyle-styrène.

Avant d'utiliser le coussin thermique des figures 1 à 3 dans une application thérapeutique, on le maintient, par exemple dans une armoire frigorifique, à une température suffisamment basse pour congeler la solution aqueuse des cellules 1. Le coussin thermique est retiré de l'armoire frigorifique au moment de son utilisation et on peut l'appliquer immédiatement sur une partie du corps humain que l'on souhaite soumettre à une thérapie cryogénique, par exemple une main, la tête ou un bras. Grâce à la souplesse de l'enveloppe 7 et des languettes 6 et à l'élasticité de la matière thermique 10 contenue dans les interstices 8, le coussin se déforme et s'adapte parfaitement à la morphologie du corps humain.

Dans une forme de réalisation modifiée du coussin thermique des figures 1 à 3, l'enveloppe élastique 7 est soudée aux cellules 1 et mise sous tension, de telle sorte que le corps solide élastique 10 des interstices 8 soit maintenu dans un état de compression élastique dans ces interstices 8.

Lorsque le coussin thermique représenté aux figures 1 à 3 est utilisé pour une thérapie médicale, on peut interposer une compresse entre le coussin thermique et la partie anatomique du corps humain. Toute compresse habituellement utilisée en thérapie médicale convient. Elle peut par exemple comprendre une gaze. Après avoir posé le coussin thermique sur la compresse, on le lie fermement à la partie traitée du corps, au moyen d'un bandage (adhésif ou autre), de manière qu'en se déformant, il épouse parfaitement toute la partie traitée du corps. En variante, le coussin peut être lui-même muni de parties adhésives médicales de type repositionnables. Dans ce cas particulier, le bandage est superflu.

Pour améliorer l'action thermique du coussin sur la partie anatomique traitée, on peut, avec avantage, poser un second coussin thermique sur le premier coussin thermique. Dans ce cas, seul le premier coussin thermique [celui qui est appliqué directement sur le corps humain (ou sur la compresse)] est traité dans l'armoire frigorifique, l'autre coussin thermique étant maintenu à la température ambiante. Dans cette application, le second coussin thermique peut agir comme isolant thermique. Il peut notamment faire office de bandage isolant de fixation et/ou de compression. Il peut être muni d'une poche gonflable pour l'application optimale sur des corps de forme concave.

Dans certaines formes de réalisation, le coussin peut intégrer en un article unique la fonction thermique, la fonction de bandage isolant et comprendre la compresse dont il a été question plus haut, que l'on interpose entre le coussin thermique et la partie anatomique du corps humain.

Dans une forme de réalisation modifiée du coussin thermique des figures 1 à 3, les languettes 6 sont percées d'ouvertures 9 (dont la fonction apparaîtra plus loin) et la matière thermique 10 des interstices 8 est à l'état liquide ou de gel à la température normale d'utilisation. Dans cette forme de réalisation du coussin, l'enveloppe 7 est fixée aux cellules 1, de manière à obturer hermétiquement les interstices 8. Les ouvertures 9 ont pour fonction de permettre une circulation de la matière thermique fluide dans le réseau d'interstices 8, lorsque le coussin subit une déformation.

La structure du coussin thermique permet éventuellement son utilisation par circulation de fluide en interstices et/ou en cellules (communicantes par canaux de remplissage/circulation).

Des réactions de mélanges thermogènes/cryogènes peuvent être utilisées pour initier la fonction thermique de manière autonome ou pour prolonger la durée d'action sans nécessiter la dépose pour recharge du coussin.

Le coussin peut comporter des éléments de contrôle et régulation de température.

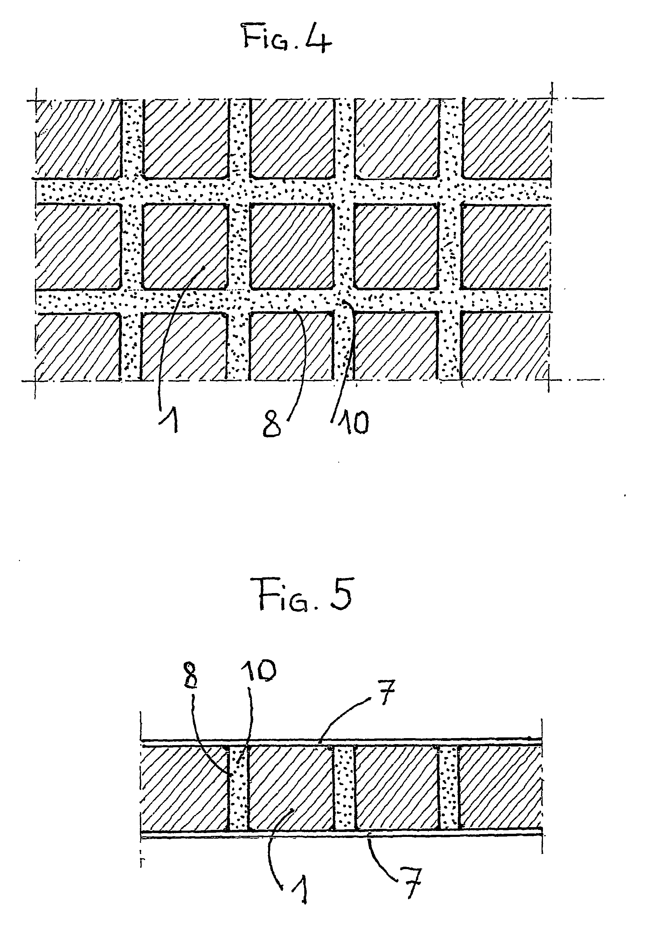

Dans la forme de réalisation représentée aux figures 4 et 5, le coussin comprend un réseau de cellules 1 entre lesquelles est interposé un réseau d'une masse solide élastique 10. La masse élastique solide 10 est par exemple une masse en élastomère ou une mousse en résine polymèrique. Le réseau élastique 10 est fixé au réseau de cellules 1 par collage ou soudage. Le réseau élastique 10 sert ainsi d'articulation entre les cellules 1.

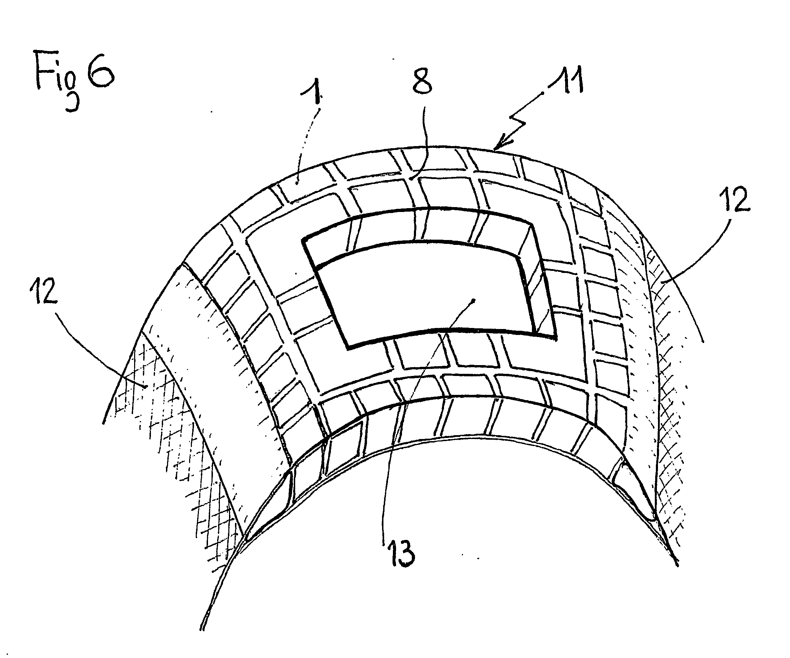

La figure 6 montre l'utilisation du coussin thermique selon l'invention dans un dispositif pour le traitement thérapeutique d'un élément du corps humain. Le traitement thérapeutique peut par exemple consister en un traitement cryogénique d'un bras ou d'une cuisse. A cet effet, le dispositif comprend un coussin thermique désigné dans son ensemble par la notation de référence 11. Le coussin thermique comprend, comme exposé plus haut, un réseau de blocs 1 et d'interstices 8, contenant des matières thermiques appropriées. Le coussin thermique 11 est fixé à un bandage 12, destiné à l'attacher au bras d'une personne. Une ouverture rectangulaire 13 est enclavée dans le réseau de blocs 8. L'ouverture 13 sert à donner accès à une zone définie du bras, pour soumettre celle-ci à un traitement thérapeutique approprié (par exemple y traiter une plaie, une contusion ou une autre affection locale). L'ouverture 13 peut être utilisée pour appliquer un pansement sur le bras. En variante, elle peut être comblée par un petit coussin thermique amovible, conforme à l'invention. Le coussin thermique 11 du dispositif de la figure 6 peut par exemple être un coussin cryogénique, conçu pour mettre le bras traité en hypothermie pendant qu'on soumet la zone accessible dans l'ouverture 13 à un traitement thérapeutique approprié.

Le dispositif de la figure 6 peut évidemment être adapté au traitement d'un membre du corps d'un animal, par exemple une patte d'un cheval.

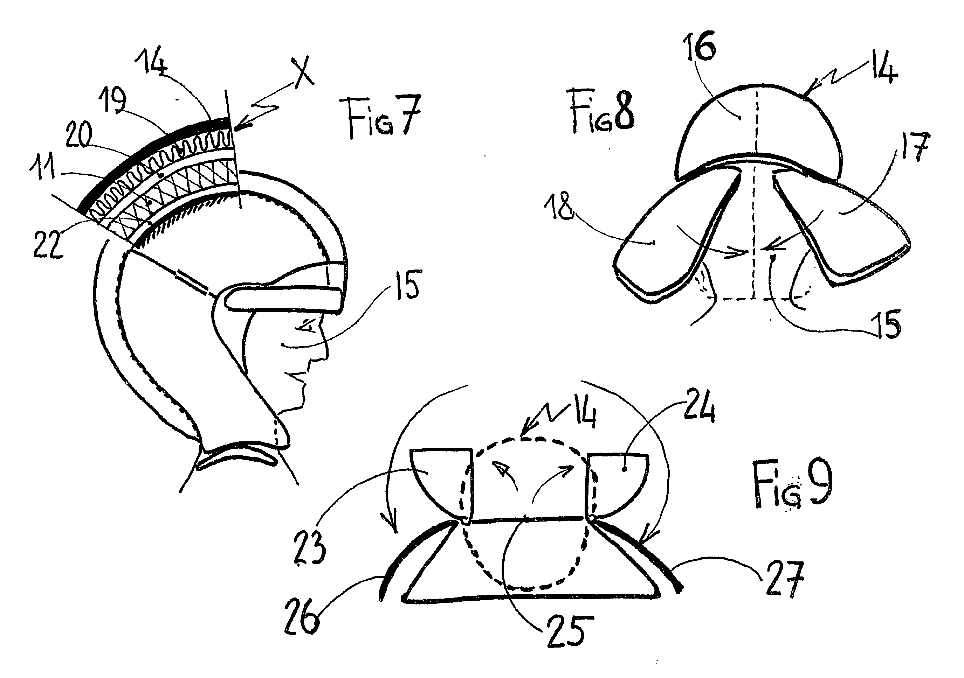

Les figures 7 et 8 montrent un dispositif conforme à l'invention pour le traitement thérapeutique du crâne d'une personne. Le dispositif comprend une coquille rigide 14, ayant la forme d'un casque épousant sensiblement la tête 15 et le cou de la personne. Le casque 14 est formé de trois éléments articulés 16, 17 et 18 (figure 8). L'élément 16 sert à recouvrir la calotte crânienne et les éléments 17 et 18 sont destinés à encercler le cou. La partie intérieure du casque 14 est représentée à plus grande échelle dans une zone désignée par la notation de référence X à la figure 7. Celle-ci comprend un coussin thermique, sous la coquille 14. Le coussin thermique est conforme à l'invention et comprend successivement une couche isolante 19, une enveloppe étanche 20 (dont la fonction sera explicitée plus loin), un réseau 11 de blocs articulés contenant une matière thermique et une seconde enveloppe étanche 22. L'enveloppe étanche 20 comprend un dispositif (non représentée) pour y introduire un fluide sous pression. Il s'agit généralement d'air que l'on insuffle dans la poche au moyen d'une pompe électrique ou manuelle (du type des pompes pour bicyclettes ou utilisées sur les tensiomètres médicaux ou paramédicaux). La poche 22 est similaire à la poche 20 est également conçue pour l'introduction d'un volume défini d'air sous pression. Lorsqu'il s'agit de soumettre la tête d'un patient à un traitement cryogénique, par exemple la placer en hypothermie, on maintient le casque 14 et son coussin thermique dans une armoire frigorifique pendant un temps approprié pour qu'ils acquièrent une température prédéterminée. On applique ensuite l'élément 16 du casque 14 sur la calotte crânienne de la tête 15 du patient et on rabat les éléments 17 et 18 contre le cou du patient. On insuffle par la suite un volume défini d'air dans la poche 20, pour appliquer le réseau de blocs articulés 11 avec une pression prédéterminée sur la tête 15 du patient. Par la suite, on insuffle éventuellement un volume défini d'air dans la poche 22. La poche d'air 22 sert à former un film isolant entre le réseau de blocs 11 et la tête 15, pour réguler le flux de chaleur entre la tête 15 et la matière thermique des blocs 11 du coussin thermique. On régule à volonté ce flux de chaleur par un choix approprié du volume d'air que l'on insuffle dans la poche 22.
La coquille 14 du dispositif des figures 7 et 8 peut avantageusement être munie d'une instrumentation médicale, comprenant par exemple des thermomètres ou des tensiomètres.

La figure 9 montre une variante de réalisation du dispositif des figures 7 et 8. Dans le dispositif de la figure 9, la coquille 14 a la forme d'un hémisphère, adapté à recouvrir la calotte crânienne d'une personne. Elle est formée de deux éléments 23 et 24 articulés sur un anneau 25 destiné à encercler la boîte crânienne à hauteur du front. Les éléments 23 et 24 portent, sur leur face interne, un coussin thermique (non représenté), conforme à l'invention. Deux panneaux 26 et 27 sont par ailleurs articulés à l'anneau 25. Les deux panneaux 26 et 27 forment, avec les éléments 23 et 24, une chambre dans laquelle on enferme les coussins thermiques des éléments 23 et 24. La coquille 14 et ses coussins thermiques font ainsi partie intégrante d'un caisson (qui peut être hermétique et isolant) pour le transport et la manutention du dispositif. Le dispositif représenté à la figure 9 est spécialement bien adapté à une utilisation en urgence, en dehors d'un centre hospitalier, par exemple pour intervenir en urgence sur la voie publique. La coquille 14 peut en outre être munie d'un appareil autonome pour générer de la chaleur ou du froid, pendant le transport du dispositif ou sa manutention.

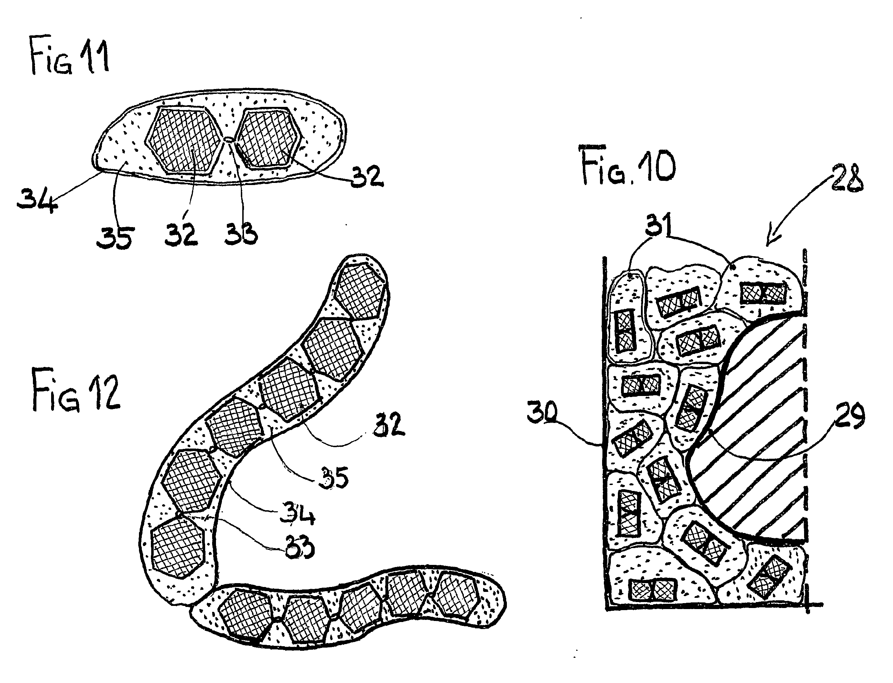

La figure 10 montre une autre application du coussin thermique selon l'invention. Elle montre partiellement une paroi cryogénique 28 d'un thermos, destiné à conserver des organes ou des aliments froids. La paroi cryogénique 28 comprend une paire de cloisons 29 et 30, délimitant entre-elles une chambre remplie de petits coussins cryogéniques 31 conformes à l'invention. Conformément à l'invention, chaque coussin cryogénique 31 comprend des blocs 32 (figure 11) reliés par une membrane souple 33 et remplis d'une substance cryogénique rigide, par exemple de la glace. Le réseau des blocs 32 est enfermé dans une enveloppe souple et hermétique 34, remplie d'une matière cryogénique fluide 35. La matière cryogénique fluide 35 peut par exemple comprendre un gel. Le caractère déformable de la matière cryogénique 35 optimise les propriétés réfrigérantes de la paroi 28.

La figure 12 montre une autre forme de réalisation des coussins cryogéniques 31 utilisables dans la paroi cryogénique 28 de la figure 10. Dans cette forme de réalisation, le coussin cryogénique comprend une chaîne de blocs 32 reliés par des pivots 33 et contenant une matière cryogénique rigide (par exemple de la glace). La chaîne des blocs 32 est enfermée dans une enveloppe tubulaire 34, remplie d'un gel cryogénique 35.

Par extension, l'invention concerne aussi les parois thermiques dans lesquelles les coussins thermiques contiennent un bloc unique 32 (contenant une matière thermique rigide), dans une enveloppe souple contenant une matière thermique fluide [liquide, gaz, gel ou solide à l'état de particules meubles (poudre)].

## Revendications

1. Coussin thermique comportant un réseau de blocs reliés par des articulations et séparés par des interstices, lesdits blocs comprenant une matière thermique et lesdits interstices étant comblés, au moins partiellement, avec une matière thermique déformable, **caractérisé en ce que** les articulations sont sélectionnées parmi
- les corps solides élastiques qui sont fixés aux blocs (1) et constituent alors une partie au moins de la matière thermique déformable susdite ;
- les membranes élastiques (7), qui sont fixées aux blocs (1) et mises sous tension de manière à comprimer un corps déformable, présent dans les interstices (8), ledit corps déformable constituant alors une partie au moins de la matière thermique déformable ; et
- les articulations (6), qui sont perméables à un fluide présent dans les interstices (8), ledit fluide constituant alors une partie au moins de la matière thermique déformable.

2. Coussin selon la revendication 1, **caractérisé en ce que**, dans le cas où la matière thermique déformable des interstices (8) comprend un corps solide élastique, celui-ci comprend une mousse et/ou un élastomère.

3. Coussin selon la revendication 1 ou 2, **caractérisé en ce que** les articulations perméables, comprennent des membranes flexibles et/ou élastiques et perforées (9).

4. Coussin selon l'une quelconque des revendications 1 à 3, **caractérisé en ce qu'**une feuille souple et/ou élastique (7) enveloppe les blocs (1).

5. Coussin selon l'une quelconque des revendications 1 à 4, **caractérisé en ce que** la forme des blocs (1) est sélectionnée parmi les formes sphérique, hémisphérique, ovoïde, annulaire, lenticulaire, conique, tronconique et polyédrique.

6. Coussin selon la revendication 5, **caractérisé en ce que** les blocs (1) sont des polyèdres à base triangulaire, trapézoïdale, carrée ou octogonale.

7. Coussin selon la revendication 6, **caractérisé en ce que** chaque bloc (1) est formé de deux troncs de pyramide (3, 4), qui sont joints le long de leur grande base et **en ce que** les blocs sont articulés sur une articulation (6) qui est disposée dans le plan géométrique de ladite grande base.

8. Coussin selon l'une quelconque des revendications 1 à 7, **caractérisé en ce que** les blocs (1) comprennent des cellules contenant la matière thermique.

9. Coussin selon la revendication 8, **caractérisé en ce que**, dans le cas où la matière thermique des blocs (1) présente un état liquide ou gazeux, les cellules sont étanches audit état de la matière thermique.

10. Coussin thermique selon l'une quelconque des revendications 1 à 9, **caractérisé en ce que** l'une au moins des matières thermiques comprend une substance qui subit un changement d'état à la température normale d'utilisation.

11. Coussin selon la revendication 10, **caractérisé en ce que** la substance susdite est un corps pur ou une composition chimique qui est congruente à la température normale d'utilisation.

12. Coussin thermique selon l'une quelconque des revendications 1 à 11, **caractérisé en ce que** le réseau de blocs (11) est recouvert d'une enveloppe élastique et étanche (20 ou 22), munie de dispositif d'admission et échappement d'un fluide.

13. Coussin selon l'une quelconque des revendications 1 à 11, **caractérisé en ce qu'**une ouverture (13) est enclavée dans le réseau de blocs (11).

14. Coussin selon la revendication 13, **caractérisé en ce que** l'ouverture (13) contient un autre réseau de blocs, amovible.

15. Coussin selon l'une quelconque des revendications 1 à 11, 13 et 14, **caractérisé en ce que** les blocs du réseau sont articulés en une seule strate.

16. Coussin selon la revendication 15, **caractérisé en ce que** la strate est prise en sandwich entre deux enveloppes élastiques et étanches (20, 22), munies chacune d'un dispositif d'admission et échappement d'un fluide.

17. Coussin selon l'une quelconque des revendications 1 à 11, 13 et 14, **caractérisé en ce que** les blocs du réseau sont disposés en au moins deux strates.

18. Coussin selon la revendication 17, **caractérisé en ce que** les deux strates sont prises en sandwich entre deux enveloppes élastiques et étanches, munies chacune d'un dispositif d'admission et d'échappement d'un fluide.

19. Coussin selon l'une quelconque des revendications 1 à 11, 15 et 17, **caractérisé en ce que** le réseau de blocs est disposé dans une enveloppe comblée d'une matière thermique déformable.

20. Coussin selon la revendication 19, **caractérisé en ce qu'**une partie au moins de la matière thermique de l'enveloppe comprend la matière thermique des interstices.

21. Dispositif comprenant un coussin thermique conforme à l'une quelconque des revendications 1 à 20, pour un traitement thérapeutique du corps humain ou animal.

22. Dispositif selon la revendication 21, **caractérisé en ce que** le coussin (11) est logé à l'intérieur d'une coquille rigide (14).

23. Dispositif selon la revendication 22, **caractérisé en ce que** la coquille rigide (14) a la forme d'un crâne humain (15).

24. Dispositif selon la revendication 22 ou 23, **caractérisé en ce qu'**une enveloppe élastique et étanche (20) est interposée entre le réseau de blocs (11) et la coquille (14), ladite enveloppe étant munie d'un dispositif d'admission et échappement d'un fluide.

25. Dispositif selon l'une quelconque des revendications 22 à 24, **caractérisé en ce que** la coquille (14) comprend au moins deux éléments articulés (16, 17, 18).

26. Dispositif selon l'une quelconque des revendications 22 à 25, **caractérisé en ce qu'**au moins un panneau (26, 27) est articulé à la coquille (14) et dimensionné en sorte de pouvoir former, avec la coquille, une chambre sensiblement hermétique contenant le coussin.

27. Dispositif selon la revendication 26, **caractérisé en ce que** la coquille (14) et/ou un panneau (26,27) comprend une instrumentation médicale.

28. Paroi thermique comprenant une accumulation de coussins thermiques (31) conformes à la revendication 19 ou 20, entre deux cloisons (29, 30).

## Claims

1. Thermal cushion comprising a network of blocks connected by articulations and separated by interstices, said blocks comprising a thermal substance and said interstices being filled, at least partially, with a deformable thermal substance, **characterised in that** the articulations are selected from
- the elastic solid bodies that are attached to the blocks (1) and thus form at least part of the above-mentioned deformable thermal substance;
- the elastic membranes (7) that are attached to the blocks (1) and put under tension so as to compress a deformable body present in the interstices (8), said deformable body forming at least part of the deformable thermal substance; and
- the articulations (6) that are permeable to a fluid present in the interstices (8), said fluid thus forming at least part of the deformable thermal substance.

2. Cushion according to Claim 1, **characterised in that**, in the case where the deformable thermal substance of the interstices (8) comprise an elastic solid body, the latter comprises a foam and/or an elastomer.

3. Cushion according to Claim 1 or 2, **characterised in that** the permeable articulations comprise perforated, flexible and/or elastic membranes (9).

4. Cushion according to any one of Claims 1 to 3, **characterised in that** a flexible and/or elastic sheet (7) wraps the blocks (1).

5. Cushion according to any one of Claims 1 to 4, **characterised in that** the shape of the blocks (1) is selected from spherical, hemispherical, ovoid, annular, lenticular, conical, truncated conical and polyhedral shapes.

6. Cushion according to Claim 5, **characterised in that** the blocks (1) are polyhedrons with a triangular, trapezoidal, square or octagonal base.

7. Cushion according to Claim 6, **characterised in that** each block (1) is formed by two truncated pyramids (3, 4) joined along their long bases and **in that** the blocks are articulated on a articulation (6) positioned in the geometrical plane of said long base.

8. Cushion according to any one of Claims 1 to 7, **characterised in that** the blocks (1) comprise cells with the thermal substance.

9. Cushion according to Claim 8, **characterised in that**, in the case where the thermal substance of the blocks (1) is in a liquid or gaseous state, the cells are sealed relative to the state of the thermal substance.

10. Thermal cushion according to any one of Claims 1 to 9, **characterised in that** at least one of the thermal substances comprises a substance that is subjected to a change of state at normal temperature of use.

11. Cushion according to Claim 10, **characterised in that** the above-mentioned substance is a pure body or a chemical compound that is congruent at normal temperature of use.

12. Thermal cushion according to any one of Claims 1 to 11, **characterised in that** the network of blocks (11) is covered by a sealed and elastic envelope (20 or 22) provided with an intake and outlet device for a fluid.

13. Cushion according to any one of Claims 1 to 11, **characterised in that** an aperture (13) is made in the network of blocks (11).

14. cushion according to claim 13, **characterised in that** the aperture (13) comprises another, removable network of blocks.

15. Cushion according to any one of Claims 1 to 11, 13 and 14, **characterised in that** the blocks of the network are articulated in a single layer.

16. Cushion according to Claim 15, **characterised in that** the layer is maintained in a sandwich between two sealed and elastic envelopes (20,22), being each provided with a device for the intake and outlet of a fluid.

17. Cushion according to any one of Claims 1 to 11, 13 and 14, **characterised in that** the blocks of the network are arranged in at least two layers.

18. Cushion according to Claim 17, **characterised in that** the two layers are maintained in a sandwich between two sealed and elastic envelopes, being each provided with a device for the intake and outlet of a fluid.

19. Device according to any one of Claims 1 to 11, 15 and 17, **characterised in that** the network of blocks is arranged in an envelope filled with a deformable thermal substance.

20. Cushion according to Claim 19, **characterised in that** at least part of the thermal substance of the envelope comprises the thermal substance of the interstices.

21. Device comprising a thermal cushion according to any one of Claims 1 to 20 for the therapeutic treatment of a human or animal body.

22. Device according to Claim 21, **characterised in that** the cushion (11) is placed inside a rigid shell (14).

23. Device according to Claim 22, **characterised in that** the rigid shell (14) takes the shape of a human skull (15).

24. Device according to Claim 22 or 23, **characterised in that** a sealed and elastic envelope (20) is inserted between the network of blocks (11) and the shell (14), said envelope being provided with a device for the intake and outlet of a fluid.

25. Device according to any one of Claims 22 to 24, **characterised in that** the shell (14) comprises at least two articulated elements (16,17,18).

26. Device according to any one of Claims 22 to 25, **characterised in that** at least one panel (26,27) is articulated to the shell (14) and sized so as to form, together with the shell, a chamber that is more or less hermetic and comprises the cushion.

27. Device according to Claim 26, **characterised in that** the shell (14) and/or a panel (26,27) comprises medical tools.

28. Thermal wall comprising an assembly of thermal cushions (31) according to Claim 19 or 20, between two partitions (29,30).

## Patentansprüche

1. Heizkissen, bestehend aus einem Netzwerk aus Blöcken, die über Gelenkstücke miteinander verbunden und durch Zwischenräume voneinander getrennt sind; die besagten Blöcke enthalten ein thermisches Material, während die besagten Zwischenräume zumindest teilweise mit einem verformbaren thermischen Material aufgefüllt sind. Das Kissen ist **dadurch gekennzeichnet, dass** die Gelenkstücke aus den folgenden Möglichkeiten ausgewählt werden :
- Elastische, an den Blöcken (1) befestigte feste Körper, die somit zumindest einen Teil des besagten verformbaren thermischen Materials darstellen;
- Elastische Membranen (7), die an den Blöcken (1) befestigt und so unter Spannung gebracht werden, dass sie einen verformbaren Körper, der in den Zwischenräumen (8) vorhanden ist, zusammendrücken; der besagte verformbare Körper stellt somit zumindest einen Teil des verformbaren thermischen Materials dar; und
- Die Gelenkstücke (6), die gegenüber einer in den Zwischenräumen (8) vorhandenen Flüssigkeit durchlässig sind; die besagte Flüssigkeit stellt somit zumindest einen Teil des verformbaren thermischen Materials dar.

2. Kissen entsprechend dem Anspruch 1, **dadurch gekennzeichnet, dass** für den Fall, in dem das verformbare thermische Material der Zwischenräume (8) einen festen elastischen Körper umfasst, dieser Körper einen Schaum und/oder ein Elastomer enthält.

3. Kissen entsprechend den Ansprüchen 1 oder 2, **dadurch gekennzeichnet, dass** die durchlässigen Gelenkstücke flexible und/oder elastische sowie perforierte Membranen(9) enthalten.

4. Kissen gemäß einem der Ansprüche 1 bis 3, **dadurch gekennzeichnet, dass** eine weiche und/oder elastische Folie (7) die Blöcke (1) umhüllt.

5. Kissen gemäß einem der Ansprüche 1 bis 4, **dadurch gekennzeichnet, dass** die Blöcke (1) in einer kugel-, halbkugel-, ei-, ring-, linsen-, kegel-, kegelstumpfförmigen und vielflächigen Form gewählt werden.

6. Kissen entsprechend dem Anspruch 5, **dadurch gekennzeichnet, dass** es sich bei den Blöcken (1) um Vielecke mit dreieckiger, trapezförmiger, viereckiger oder achteckiger Grundfläche handelt.

7. Kissen entsprechend dem Anspruch 6, **dadurch gekennzeichnet, dass** jeder Block (1) aus zwei Pyramidenstümpfen {3, 4) gebildet wird, die entlang ihrer großen Grundfläche verbunden sind, und **dadurch gekennzeichnet, dass** die Blöcke über ein Gelenkstück (6) beweglich verbunden sind, das in der geometrischen Fläche der besagten großen Grundfläche angeordnet ist.

8. Kissen gemäß einem der Ansprüche 1 bis 7, **dadurch gekennzeichnet, dass** die Blöcke (1) Zellen enthalten, die wiederum das thermische Material enthalten.

9. Kissen gemäß dem Anspruch 8, **dadurch gekennzeichnet, dass** in dem Fall, in dem das thermische Material der Blöcke (1) in flüssigem oder gasförmigem Zustand vorliegt, die Zellen gegenüber dem besagten thermischen Zustand des thermischen Materials abgedichtet sind.

10. Kissen entsprechend einem der Ansprüche 1 bis 9, **dadurch gekennzeichnet, dass** zumindest eines der thermischen Materialien eine Substanz enthält, die bei normaler Nutzungstemperatur einer Zustandsänderung ausgesetzt ist.

11. Kissen entsprechend dem Anspruch 10, **dadurch gekennzeichnet, dass** es sich bei der besagten Substanz um einen reinen Körper bzw. um eine chemische Zusammensetzung handelt, die mit der "normalen Nutzungstemperatur" übereinstimmt.

12. Heizkissen entsprechend einem der Ansprüche 1 bis 11, **dadurch gekennzeichnet, dass** das Netz der Blöcke (11) durch eine elastische und dichte Hülle (20 bzw. 22) abgedeckt ist, die mit einer Vorrichtung zum Ein- und Auslassen einer Flüssigkeit ausgestattet ist.

13. Kissen gemäß einem der Ansprüche 1 bis 11, **dadurch gekennzeichnet, dass** im Netz der Blöcke (11) eine Öffnung (13) eingefügt ist.

14. Kissen entsprechend dem Anspruch 13, **dadurch gekennzeichnet, dass** diese Öffnung (13) ein weiteres, herausnehmbares Netz von Blöcken enthält.

15. Kissen entsprechend einem der Ansprüche 1 bis 11, 13 und 14, **dadurch gekennzeichnet, dass** die Blöcke des Netzes in einer einzigen Schicht durch Gelenke miteinander verbunden sind.

16. Kissen entsprechend dem Anspruch 15, **dadurch gekennzeichnet, dass** diese Schicht in der Mitte zwischen zwei elastischen und dichten Hüllen (20, 22) angeordnet ist, die jeweils mit einer Vorrichtung für das Ein- und Auslassen einer Flüssigkeit ausgestattet sind.

17. Kissen entsprechend einem der Ansprüche 1 bis 11, 13 und 14, **dadurch gekennzeichnet, dass** die Blöcke des Netzes in mindestens zwei Schichten angeordnet sind.

18. Kissen entsprechend dem Anspruch 17, **dadurch gekennzeichnet, dass** diese zwei Schichten in der Mitte zwischen zwei elastischen und dichten Hüllen angeordnet sind, die jeweils mit einer Vorrichtung für das Ein- und Auslassen einer Flüssigkeit ausgestattet sind.

19. Kissen entsprechend einem der Ansprüche 1 bis 11, 15 bis 17, **dadurch gekennzeichnet, dass** das Netz der Blöcke in einer mit einem verformbaren thermischen Material aufgefüllten Hülle angeordnet ist.

20. Kissen gemäß Anspruch 19, **dadurch gekennzeichnet, dass** mindestens ein Teil des thermischen Hüllenmaterials das thermische Material der Zwischenräume enthält.

21. Vorrichtung, die ein Heizkissen entsprechend einem der Ansprüche 1 bis 20 enthält und die der therapeutischen Behandlung des Körpers eines Menschen oder eines Tieres dient.

22. Vorrichtung entsprechend dem Anspruch 21, **dadurch gekennzeichnet, dass** das Kissen (11) im Inneren einer harten Schale (14) gelagert wird.

23. Vorrichtung entsprechend dem Anspruch 22, **dadurch gekennzeichnet, dass** die feste Schale (14) die Form eines menschlichen Schädels (15) hat.

24. Vorrichtung entsprechend den Ansprüchen 22 bzw. 23, **dadurch gekennzeichnet, dass** zwischen dem Netz der Blöcke (11) und der Schale (14) eine elastische und dichte Hülle (20) eingefügt wird; die besagte Hülle ist mit einer Vorrichtung zum Ein- und Auslassen einer Flüssigkeit ausgestattet.

25. Vorrichtung entsprechend einem der Ansprüche 22 bis 24, **dadurch gekennzeichnet, dass** die Schale (14) mindestens zwei miteinander beweglich verbundene Elemente(16, 17, 18) enthält.

26. Vorrichtung entsprechend einem der Ansprüche 22 bis 25, **dadurch gekennzeichnet, dass** mindestens eine Platte (26, 27) mit der Schale (14) beweglich verbunden und derart dimensioniert ist, dass sie mit der Schale eine spürbar luftdichte Kammer bildet, die das Kissen enthält.

27. Vorrichtung gemäß dem Anspruch 26, **dadurch gekennzeichnet, dass** die Schale (14) und/oder die Platte (26, 27) ein medizinisches Instrument enthalten.

28. Thermische Wand, die eine Anhäufung thermischer Kissen (31) entsprechend dem Anspruch 19 bzw. 20 zwischen zwei Trennwänden (29, 30) enthält.
